# EUROPEAN PATENT SPECIFICATION

(11) **EP 2 598 205 B1**
(45) Date of publication and mention of the grant of the patent: **31.08.2016**
(21) Application number: 11770513.7
(22) Date of filing: 29.07.2011
(51) Int. Cl.: A61N 5/10, A61N 7/02

(54) **MOTION COMPENSATION FOR NON-INVASIVE TREATMENT THERAPIES**
BEWEGUNGSKOMPENSATION FÜR NICHT-INVASIVE BEHANDLUNGSTHERAPIEN
COMPENSATION DE MOUVEMENT POUR DES THÉRAPIES DE TRAITEMENT NON INVASIVES

(30) Priority: 29.07.2010 US 368895 P
(43) Date of publication of application: 05.06.2013
(73) Proprietor: Insightec Ltd., 39120 Tirat-Carmel (IL)
(72) Inventor: VORTMAN, Kobi, 34467 Haifa (IL); ZADICARIO, Eyal, Tel-Aviv-Yafo (IL); VITEK, Shuki, 34467 Haifa (IL)
(74) Representative: Atkinson, Jonathan David Mark
(86) International application number: PCT/IB2011/002117
(87) International publication number: WO 2012/014074

(56) References cited:
- WO-A1-2007/084272
- US-A1- 2002 077 545
- US-A1- 2009 124 893

## Description

### TECHNICAL FIELD

The present invention relates generally to systems for performing noninvasive procedures using acoustic energy, and, more particularly, to systems for focusing and adjusting the delivery of ultrasonic energy during treatment.

### BACKGROUND

Tissue, such as a benign or malignant tumor or blood clot within a patient's skull or other body region, may be treated invasively by surgically removing the tissue or non-invasively by using, for example, thermal ablation. Both approaches may effectively treat certain localized conditions within the brain, but involve delicate procedures to avoid destroying or damaging otherwise healthy tissue. Unless the healthy tissue can be spared or its destruction is unlikely to adversely affect physiological function, surgery may not be appropriate for conditions in which diseased tissue is integrated into healthy tissue.

Thermal ablation, as may be accomplished using focused ultrasound, has particular appeal for treating diseased tissue surrounded by or neighboring healthy tissue or organs because the effects of ultrasound energy can be confined to a well-defined target region. Ultrasonic energy may be focused to a zone having a cross-section of only a few millimeters due to relatively short wavelengths (e.g., as small as 1.5 millimeters (mm) in cross-section at one Megahertz (1 MHz)). Moreover, because acoustic energy generally penetrates well through soft tissues, intervening anatomy often does not impose an obstacle to defining a desired focal zone. Thus, ultrasonic energy may be focused at a small target in order to ablate diseased tissue without significantly damaging surrounding healthy tissue.

To focus ultrasonic energy toward a desired target, drive signals may be sent to a piezoelectric transducer having a number of transducer elements such that constructive inference occurs at the focal zone. At the target, sufficient acoustic energy may be delivered to heat tissue until necrosis occurs, i.e., until the tissue is destroyed. Preferably, tissue along the path through which the acoustic energy passes (the "pass zone") outside the focal zone is heated only minimally, if at all, thereby minimizing damaging tissue outside the focal zone.

However, because the human body is flexible and moves (due to breathing, for example), treatment delivered as multiple sonications over time- even when delivered within seconds of each other- may require interim adjustments to targeting and/or to one or more treatment parameters. Indeed, absorption of ultrasound energy may itself change the shape and/or location of the target through swelling, for example, necessitating similar changes. This creates a significant challenge given the need to avoid damage to healthy tissue while still achieving complete ablation of the target. Tissue movement monitorring is discussed in e.g. document US 2002/0077545.

Accordingly, there is a need for systems and methods for effectively focusing acoustic energy in a manner that does not adversely affect surrounding tissue and can be administered in a timely fashion while considering morphology changes and movement of both the target and the surrounding tissue.

### SUMMARY

The present invention provides systems that facilitate non-invasive, focused delivery of ultrasound energy to a target tissue, typically for the purpose of destroying the target (e.g., through ablation) while sparing adjacent tissue. In general, the technique uses a closed-loop feedback approach that tracks and addresses anatomical movement and/or morphology changes during treatment. Various aspects of the present invention involve obtaining three-dimensional (3D) images of the treatment region (i.e., the target and surrounding tissues) and parsing the image into "voxels"- i.e., volumetric pixels- with which various attributes can be associated. Such attributes include, a maximum dosage that can be tolerated by the tissue represented by the voxel and/or a minimum dosage required to ablate the tissue. A voxel may, for example, lie in the target zone (i.e., the region intended to be treated) and require a full thermal dose, or outside the target zone, where dose tolerance depends on the type of tissue. Movement of the patient's anatomy may be tracked on a global basis (i.e., for the treatment region as a whole), and the coordinates of each voxel are updated based on the global anatomical motion. Alternatively, movement may be tracked directly at the voxel level. Based on the updated voxel coordinates, the delivered treatment is then tailored to the anatomy as it exists at that time, rather than as it previously existed.

In a first aspect, not forming part of the invention, a method of monitoring movement of a volume of tissue to facilitate treatment of the tissue is disclosed. The method involves computationally identifying voxels corresponding to the volume of tissue within a voxel coordinate space prior to movement of the tissue, and associating treatment-related attributes with at least some of the voxels in computer memory. The treatment-related attributes may include whether a voxel is a target voxel, a low-energy-tolerant voxel, or a no-pass voxel. Further, they may include optimal, maximum tolerable, and/or minimum effective temperatures and/or energy dosages. In some embodiment, the attributes include information about a treatment history of the voxels, for example, whether a voxel was treated successfully in a previous treatment dose.

The method further involves determining parameters characterizing movement of the tissue. The movement-characterizing parameters may be determined by tracking anatomical landmarks within the volume of tissue, and/or by performing image-based correlation on a series of temporally displaced images of the volume of tissue. The parameters may characterize rigid as well as non-rigid motion. Based on the determined parameters, the positions of the voxels are computationally shifted within the voxel coordinate space. One or more treatment parameters (such as, e.g., the position of an energy source relative to the tissue, or the intensity or dose of energy to which the tissue is exposed during treatment) are then altered based on the treatment-related attributes of the shifted voxels. The method includes sampling image data received over time, and averaging voxel attribute data corrected for motion, thus improving a signal-to-noise ratio associated with the image data.

In a first aspect of the invention, the invention is directed to a system for monitoring movement of a volume of tissue within a patient to facilitate treatment, according to claim 1. The system includes a memory (such as, e.g., a register, cache memory, random-access memory, or mass storage device) for storing one or more three-dimensional images of the volume of tissue. Further, the system includes a processor for (i) identifying voxels within the volume based on the images, (ii) assigning voxel- space coordinates to the voxels prior to movement of the tissue, (iii) associating treatment- related attributes with at least some of the voxels, (iv) determining parameters characterizing movement of the tissue, (iv) determining shifted positions of the voxels within the voxel coordinate space based on the parameters, and (v) altering at least one treatment parameter based on the attributes of the shifted voxels. The system may also include a data storage module for storing the voxel-space coordinates and the treatment-related attributes associated with the voxels. Further, the system may include an imaging device for obtaining the three-dimensional images. In some embodiments, the system includes an ultrasound transducer for delivering ultrasound energy to the treatment volume, and a controller for adjusting the delivery of ultrasound energy from the transducer based on the altered treatment parameters.

In a second aspect, not forming part of the invention, a method of treating a volume of tissue by exposure of the tissue to energy from a source (e.g., by applying focused ultrasound to the tissue) is disclosed. The method involves identifying, within a voxel coordinate space, voxels corresponding to the volume of tissue, and associating treatment-related attributes with at least some of the voxels (e.g., by specifying whether a voxel is a target voxel, a low-energy-tolerant voxel, or a no-pass voxel). Further, the method includes detecting movement of the tissue, characterizing the movement in three dimensions, computationally shifting positions of the voxels within the voxel coordinate space based on the characterization (the shifted voxels retaining their associated attributes), and treating the tissue based on the characterized movement and the attributes of the shifted voxels.

### BRIEF DESCRIPTION OF THE DRAWINGS

The foregoing and other objects, features and advantages of the present invention disclosed herein, as well as the invention itself, will be more fully understood from the following description of preferred embodiments and claims, when read together with the accompanying drawings, in which:
FIG. 1 is a block diagram illustrating a motion-compensation system in accordance with one embodiment; and
FIG. 2 is a schematic drawing illustrating position changes of voxels due to tissue motion in accordance with one embodiment.

### DETAILED DESCRIPTION

The present invention provides, in various embodiments, techniques and supporting systems for monitoring movement of a volume of tissue, including the target and surrounding organs and/or areas of the anatomy (hereinafter collectively referred to as the "treatment region" or "treatment volume"), to facilitate treatment of the tissue using any of a variety of non-invasive treatment modalities. The treatment region may be defined as a collection of 3D volumetric pixels, or "voxels," each of which is assigned - as an attribute associated with the voxel - an optimal (or near-optimal) dose or temperature level. The initial voxel identification and association of attributes may be performed prior to treatment, and/or during treatment in between treatment dose deliveries. Because any movement may cause the location and shape of the treatment region to change, the location of the voxels may change, both in absolute space and/or in relation to each other (due to non-rigid motion). Therefore, the originally planned dosage level and target temperature addressed to each voxel may no longer be optimal after the treatment region has moved or changed shape. To compensate for the movement, the voxels are tracked in space over time in order to redirect or refocus the treatment accordingly.

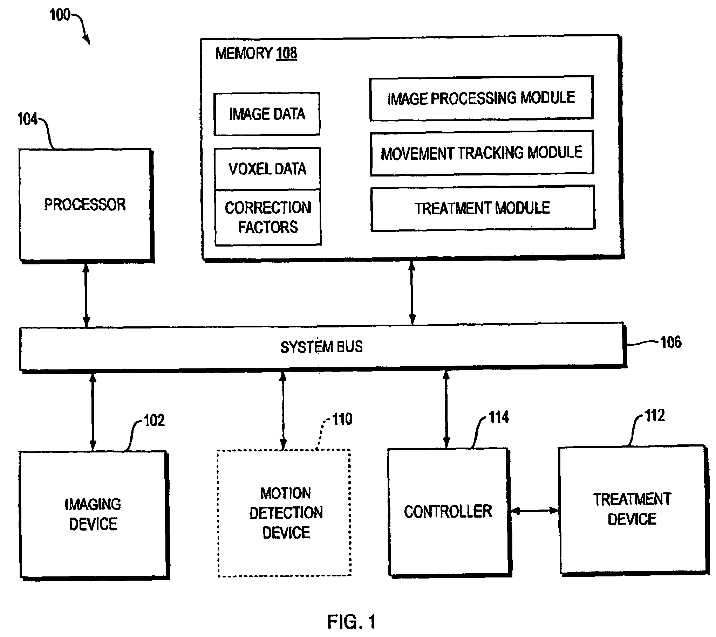

FIG. 1 illustrates an exemplary system 100 for motion compensation in accordance with various embodiments. The system 100 includes an imaging device 102, such as, for example, a magnetic resonance imaging (MRI) device, ultrasound imaging device, or computer tomography (CT) device (e.g., a positron-emission tomography (PET) system, which may be combined with X-ray computer tomography or MRI modalities). The imaging device 102 communicates with a processor 104, which may be or include, for example, the CPU of a general-purpose computer and/or a graphical processing unit (GPU), or a special-purpose microcontroller. The imaging device 102 obtains a 3D image of the treatment region, which it supplies to the processor 104 in digitized form, e.g., as bit streams sent over a system bus 106; in alternative implementations, analog signals supplied by the imaging device 102 are digitized. In some cases, the 3D image is constructed from a series of two-dimensional (2D) image slices. Further, in some embodiments, orthogonal images that provide only partial coverage of the treatment volume may be used. The 3D image data may be stored in computer memory 108, which may include registers and cache memory within the processor and main system memory (such as random-access memory (RAM)) directly accessible by the processor, as well as one or more mass storage devices (such as hard disks, optical storage media, solid-state media, etc.).

The processor 104 identifies voxels within the treatment volume, and associates with each voxel a set of coordinates in a pre-defined 3D voxel coordinate space (or "voxel space"), which may be thought of as a 3D grid of voxel-sized cubes. Each voxel, thus, corresponds to a volume element on a grid in 3D voxel space. In some embodiments, the treatment volume (or a portion thereof) is partitioned in larger-sized polyhedra instead of voxels. For example, if the tissue volume includes a significant amount of empty or homogeneously-filled space, this space may be represented by polyhedra (each of which corresponds to many voxels), while more inhomogeneous regions may be represented by individual voxels. Rigid anatomy such as bone is advantageously represented as polyhedra.

The processor 104 further assigns treatment-related attributes to the voxels. Initially, these attributes may categorize the voxels based on their location in the target region, a region outside the target region that can tolerate low energy densities (LEDR), and a no-pass zone (NPZ) that would be damaged if a treatment energy beam were to pass through it; voxels in the target region are categorized as target voxels, voxels in the LEDR are low-energy-tolerant voxels, and voxels in the NPZ are no-pass voxels. Further, the attributes may specify maximum tolerable, optimal, and/or minimal effective temperature or energy dosages for each voxel. As treatment progresses, the attributes may further include information about the treatment history of each voxels, such as an expression of the thermal dose or amount of treatment energy experienced by a voxel, or an indication whether a voxel was treated successfully (e.g., tissue therein was fully ablated) in a previous treatment dose. The data specifying the locations of the voxels in voxel coordinate space and the parameter values of attributes associated with the voxels - collectively herein referred to as "voxel data"- are stored in the memory 108, typically in the form of a database. For example, each voxel may take the form of a relational database record with associated attributes.

The imaging device 102 may take a series of temporally displaced images of the treatment volume to facilitate tracking tissue movement within the treatment region. The processor 104 analyzes these images, as described in more detail below, to determine parameters characterizing the movement with respect to the voxel coordinate space. The movement-characterizing parameters may include, for example, vectors indicating a global translation or rotation of the treatment region, relative translations between subregions, the magnitude and direction of tissue compression, distension, shearing, or other deformation (such as swelling), and/or vectors representing the translation of individual voxels. In some embodiments, the system further includes a separate movement detection device 110 that tracks movement globally, and transmits movement-characterizing parameters to the processor 104. The motion detection device 110 may be, e.g., an imaging device, laser-tracking device or similar optical system that tracks movement based on markers or fiducials placed externally on the patient, or a device implementing alternative motion-detection technologies, such as a device for detecting motion based on breathing volume and/or flow. Further, in certain embodiments, the movement may be characterized based on a computational model of movement in conjunction with image-based tracking; for example, models characterizing the morphology and behavior of particular organs are known in the art and may be used to interpret or constrain interpretation of the image data. In any case, based on the movement parameters, the processor 104 shifts the positions of the voxels within the voxel coordinate space, i.e., assigns updated coordinates to the voxels. The treatment-related attributes are shifted along with the respective voxels.

The system 100 further includes a treatment device 112, which generates an energy beam (e.g., an ultrasound or ionizing radiation beam) for heating, ablating, and/or destroying the target tissue. The beam direction, profile, and intensity, as well as the duration of energy application, may be set by a controller 114 in communication with the treatment device 112. The controller 114, in turn, may be responsive to the processor 104 so as to execute a treatment plan based on the voxel data and treatment parameters. The treatment parameters may include, for example, exposure levels, frequencies, durations, intensitites, doses, and/or other parameters that determine the exposure of the tissue to energy emitted from an energy source. In some embodiments, the treatment device 112 is a high-intensity focused ultrasound phased-array transducer that includes numerous transducer elements, each of which can deliver ultrasound energy independent of the others. In such cases, the controller 114 may include phase adjusters and amplifiers for setting the relative phases and amplitudes of the excitation signals provided to the transducer elements (or groups of transducer elements).

Upon movement of tissues within the treatment volume, the processor 104 alters various treatment parameters that affect the delivery of treatment energies to the tissue based on the newly positioned voxels and their treatment-related attributes. As will be apparent to one of skill in the art, treatment parameters may also require adjustment as a consequence of the progress of treatment, and such changes may be voxel-specific and depend on the attributes of the respective voxels. For example, following thermal dose delivery, voxels at the margin of the treatment zone may require cooling time to dissipate the heating that accumulated over time, which is not the case at target voxels. By reflecting the progress of treatment as well as tissue movement, the processor 104 facilitates the re-planning of the remaining treatment based on the new voxel locations. The controller 114 responds to the processor 104 by altering the application of acoustic energy from the transducer (via the phases, amplitudes and timing of the transducer elements), adjusting for changes in tissue conformation (e.g., the updated voxel locations of target and healthy tissues) and the updated attribute information. These adjustments to transducer operation are herein referred to as "correction factors." In certain cases, the controller 114 may also allow a human operator to manually override the computed correction factors. In some implementations, a relational or object-oriented database is used to store the voxel data (including attributes) along with the correction factors.

The computational functionality provided by the processor may be implemented by various executable program modules stored in the computer memory 108. For example, an image-processing module may identify voxels from the image data, a movement-tracking module may serve to determine parameters of movement and update the voxel coordinates based thereon, and a treatment module may adjust treatment parameters communicated to the controller 114 based on the voxel attributes, updated voxel locations and the treatment history. The program modules may be written in any of a number of suitable programming languages, including, without limitation, C, C++, C#, FORTRAN, PASCAL, Java, Tcl, or BASIC.

Controller 114 may be implemented in hardware, software or a combination of the two. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors. In typical implementations, the controller has interfaces for operating the treatment device 112, but its operation is directed by the processor 104 based on program instructions in the memory 108; in other implementations, however, the controller 114 is a dedicated device specific to, and possibly resident on, the treatment device 112, and while the processor 104 does not control the low-level operational control signals imparted by the controller 114 to the treatment device 112, its overall operation - i.e., when the treatment device should operate and the anatomical region targeted - remain subject to control of the processor 104 based on the parameters and modules described above.

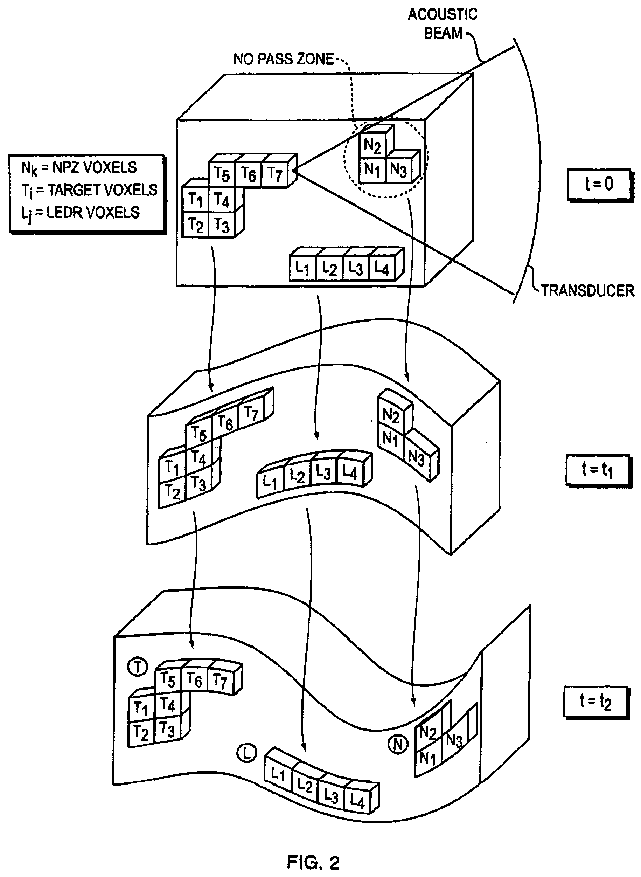

The system 100 can be used to compensate for tissue movement in a treatment setting as illustrated, for example, in FIG. 2. In this example, the treatment region is cuboid at time t = 0. By mapping the cuboid to a three-dimensional grid, a voxel coordinate space can be defined. FIG. 2 shows three groups of voxels within this space, which correspond to tissue regions in the target (i.e., at and around the focus of the energy beam), in a low energy density region (LEDR), and in a no-pass zone (NPZ), respectively. As treatment progresses, the illustrated anatomy of the treatment volume changes due, for example, to shifting of an organ, swelling, breathing, or general morphology changes. In FIG. 2, the treatment region no longer has a cuboid shape at time t = t₁, but is instead curved (i.e., bounded by curved surfaces). As a result of these morphological changes to the treatment volume, the arrangement of the voxels relative to each other has also changed. Specifically, at t = t₁, the LEDR voxels, relative to the as-yet-unmoved transducer, now intervene between the NPZ voxels and the target voxels.

The distortions and shifts - the "morphing" - in the anatomy can often be expressed by a number of global motion vectors at a macroscopic level, as well as by voxel-based vectors at the microscopic (i.e., voxel) level. One approach to characterizing motion globally involves tracking positional changes in some key anatomical landmarks or descriptors, such as tissue interfaces or organs, that can readily be identified in images of the target region, using, for example, edge-detection techniques well-known to those of skill in the art. Preferably, one or more robust anatomical descriptors, which do not deform as they move, are selected. Motion-vector analysis of the anatomical landmarks based on a directional confidence level may then be used to characterize the translation and rotation of the entire treatment volume, e.g., in terms of a matrix that can be applied to an image at t = 0 to yield the image at t = t₁. Motion vector analysis may involve encoding the displacement of each anatomical landmark with a vector, and computing global rotation and translation vectors that minimize the sum of the squares of error vectors associated with the individual displacement vectors calculated with the global vectors. To detect and characterize non-rigid motion, a weighted directional analysis, which augments non-rigid volumetric-motion vector analysis with directionally weighted contour constraints may be used. Contour constraints use knowledge of the underlying anatomy to constrain allowed movements and weight the motion vector accordingly. Once the tissue movement is characterized globally, the coordinate changes of individual voxels can readily be calculated using the global movement-characterizing parameters.

Alternatively, voxels can be tracked directly in a series of temporally-displaced 3D images of the treatment volume using an image-based correlation, i.e, by comparing the current voxel arrangement with a prior arrangement and computing movement vectors that describe the new positions for the voxels based on their prior location and their relation to other voxels within the voxel coordinate space. This approach is generally effective as long as the voxels do not move across large distances between successive images in the time series. In other words, the imaging update rate should be fast enough to allow tracking having a geometrical tracking error below a predefined threshold. For example, in some embodiments, the voxels move, between successive frames, on average by less than one voxel length (e.g., by only 0.3 voxel lengths).

By moving the shifted voxels within (or re-projecting them back into) the original voxel coordinate system, their new positions can be related to the transducer's position; consequently, the transformation may be used to adjust the treatment plan and/or transducer location and operation. In some instances, the adjustments may be made manually, whereas in other cases the adjustments may be computed as correction factors and implemented automatically using a controller, as described above.

Because the morphing operations can affect all (or a high percentage) of relevant voxels, the attributes associated with each voxel remain associated ("travel with") with the voxel as it moves to new positions in the voxel space. In other words, unlike the prior art, movements of an entire 3D area are tracked in space, so that whatever attributes are associated with points within the space travel with these points as they move. As illustrated in FIG. 2, compression and bending of the tissue from to → t₁ not only shifts the absolute positions of the NPZ, target and LEDR voxels, but also their spatial relationships to both the transducer and to each other. The different voxel types are identified, post-shifting, in the voxel grid by sorting for the characterizing attributes, facilitating re-computation of the safety envelope (given the new tissue geometry) and re-positioning the transducer accordingly. As a result, adjustments may be implemented at the voxel level in response to observed anatomical changes (e.g., patient shifts) during treatment. Computation of these shifts and updating of voxel data are accomplished by the processor 104 executing the image-processing and movement-tracking modules described above.

It may be, for example, that following transformation, certain voxels do not fit neatly within a single voxel position in the 3D grid, but instead span and partially occupy multiple voxel positions. These partly unfilled positions either inherit all or none of the attributes of the voxel that has been shifted into them (based, for example, on the degree to which the voxel occupies the position), or, if two shifted voxels now partly occupy a single position in the 3D grid, the attributes of one voxel are selected based on a set of rules stored in the memory 108 and implemented by the movement-tracking module. One such rule may be based on safety considerations (NPZ status overrides target status, for example), which voxel occupies a higher percentage of the total volume in the grid position, or which voxel occupies a particular key region of the position (e.g., nearest an organ boundary, or closest to a transition to a differently labeled region), or, in some cases, the average of the voxel attributes may be used.

In addition to allowing intra-treatment tracking, implementations of the above-described technique in conjunction with fast imaging (~10Hz, for example) results in a significant improvement in the signal-to-noise ratio (SNR) through motion-corrected summation of voxels. In general, a SNR can be improved by summing the samples because the signal will add linearly (simple summation) while the noise - assuming it is random - will add as the square root of the sum of the squares of the noise samples. For example, if the signal was measured as 3 (in some units) and the noise was measured at 2, the SNR in a single sample will be 1.5 (3/2). However, summing two samples, each with a signal value of 3 and a noise value of 2, the summed signal is 6 (3+3) while the noise value is 2.8 (√(4+4)), resulting in an SNR of 2.14 (6/2.8). Such an approach is only valid, however, if the same signal source is used for each measurement. Thus, the summation needs to be done for the same voxel while compensating for its motion. For example, if a voxel at coordinates {X1,Y1,Z1} is measured at t = to and it moves to a new location {X2, Y2, Z2}, the signal and noise values for this voxel are measured at two different locations.

## Claims

1. A system for monitoring movement of a volume of tissue within a patient to facilitate treatment thereof, the system comprising:
a memory for storing one or more three-dimensional images of the volume of tissue;
a processor configured to (i) identify voxels within the volume based on the images, (ii) assign voxel-space coordinates to the voxels prior to movement of the tissue, (iii) associate treatment-related attributes with at least some of the voxels, wherein the treatment-related attributes categorize the voxels based on their locations in a target region, a region outside the target region that tolerates low energy densities, and a no-pass zone that would be damaged if a treatment energy beam were to pass through it, voxels in the target region being categorized as target voxels, voxels in the region that tolerates low energy densities being categorized as low energy tolerant voxels, and voxels in the no-pass zone being categorized as no pass voxels, further wherein the attributes are at least one of optimal temperature, an optimal energy dosage, a maximum tolerable temperature, a maximum tolerable energy dosage, a minimum effective temperature, or a minimum effective energy dosage, (iv) determine parameters characterizing movement of the tissue, (iv) determine shifted positions of the voxels within the voxel coordinate space based on the parameters and shift positions of the voxels by assigning updated voxel-space coordinates to the voxels,, and (v) shift treatment-related attributes associated with the voxels along with the respective voxels, (vi) alter at least one treatment parameter based on new positions of the shifted voxels and the treatment-related attributes associated therewith, wherein the system is for performing non-invasive procedures using acoustic energy.

2. The system of claim 1 further comprising a data storage module for storing the voxel- space coordinates and the treatment-related attributes associated with the voxels.

3. The system of claim 1 further comprising an imaging device for obtaining the three-dimensional images.

4. The system of claim 1 further comprising an ultrasound transducer for delivering ultrasound energy to the treatment volume.

5. The system of claim 4 further comprising a controller configured to adjust the delivery of ultrasound energy from the transducer based on the altered treatment parameters.

## Patentansprüche

1. System zum Überwachen der Bewegung eines Gewebevolumens in einem Patienten, um dessen Behandlung zu erleichtern, wobei das System umfasst:
einen Speicher zum Speichern eines oder mehrerer dreidimensionaler Aufnahmen des Gewebevolumens;
einen Prozessor, ausgelegt zum (i) Identifizieren von Voxeln innerhalb des Volumens auf Grundlage der Aufnahmen, (ii) Zuweisen von Voxelraumkoordinaten an die Voxel vor einer Bewegung des Gewebes, (iii) Assoziieren behandlungsbezogener Attribute mit mindestens einigen der Voxel, wobei die behandlungsbezogenen Attribute die Voxel auf Grundlage von deren Positionen in einer Zielregion, einer Region außerhalb der Zielregion, welche niedrige Energiedichten toleriert, und einer Nicht-Durchgangs-Zone, die beschädigt werden würde, wenn ein Behandlungsenergiestrahl sie durchqueren würde, kategorisieren, wobei Voxel in der Zielregion als Zielvoxel kategorisiert werden, Voxel in der Region, die niedrige Energiedichten toleriert, als niedrigenergietolerante Voxel kategorisiert werden und Voxel in der Nicht-Durchgangs-Zone als Nicht-Durchgangs-Voxel kategorisiert werden, wobei es sich bei den Attributen ferner um mindestens eines von optimale Temperatur, eine optimale Energiedosierung, eine maximale tolerierbare Temperatur, eine maximale tolerierbare Energiedosierung, eine minimale effektive Temperatur oder eine minimale effektive Energiedosierung handelt, (iv) Bestimmen von die Bewegung des Gewebes kennzeichnenden Parametern, (iv) Bestimmen von Verschiebungspositionen der Voxel innerhalb des Voxelkoordinatenraumes auf Grundlage der Parameter und Verschiebungspositionen der Voxel durch Zuweisen aktualisierter Voxelraumkoordinaten an die Voxel und (v) Verschieben behandlungsbezogener Attribute, die mit den Voxeln assoziiert sind, nebst der entsprechenden Voxel, (vi) Ändern mindestens eines Behandlungsparameters auf Grundlage neuer Positionen der verschobenen Voxel und der damit assoziierten behandlungsbezogenen Attribute, wobei das System zum Durchführen nichtinvasiver Verfahren unter Verwendung von Schallenergie vorgesehen ist.

2. System nach Anspruch 1, ferner ein Datenspeicherungsmodul zum Speichern der Voxelraumkoordinaten und der mit den Voxeln assoziierten behandlungsbezogenen Attribute umfassend.

3. System nach Anspruch 1, ferner eine Bildgebungsvorrichtung zum Erhalten der dreidimensionalen Aufnahmen umfassend.

4. System nach Anspruch 1, ferner einen Ultraschallwandler zum Zuführen von Ultraschallenergie an das Behandlungsvolumen umfassend.

5. System nach Anspruch 4, ferner einen Regler umfassend, welcher zum Einstellen der Zuführung von Ultraschallenergie von dem Wandler auf Grundlage der geänderten Behandlungsparameter ausgelegt ist.

## Revendications

1. Un système de contrôle du mouvement d'un volume de tissu chez un patient, afin d'en faciliter le traitement, le système comprenant :
une mémoire pour le stockage d'une ou plusieurs images tridimensionnelles du volume de tissu ;
un processeur configuré pour (i) identifier des voxels au sein du volume sur la base des images, (ii) affecter des coordonnées d'espaces de voxels aux voxels préalablement au mouvement du tissu, (iii) associer des attributs relatifs au traitement avec au moins certains des voxels, les attributs relatifs au traitement classant les voxels en fonction de leur emplacement dans une région cible, une région hors de la région cible tolérant des densités à faible énergie, et une zone passe pas qui serait endommagée si elle était traversée par un faisceau d'énergie de traitement, les voxels dans la région cible étant classés comme des voxels cible, les voxels dans la région tolérant les densités à faible énergie étant classées comme des voxels tolérant de faibles énergies, et les voxels dans la zone passe pas étant classés comme des voxels passe pas, les attributs présentant au minimum un des attributs que sont une température optimale, une dose d'énergie optimale, une température tolérable maximale, une dose d'énergie tolérable maximale, une température efficace minimale, ou une dose d'énergie efficace minimale, (iv) déterminer des paramètres caractérisant le mouvement du tissu, (iv) déterminer des positions déplacées des voxels au sein de l'espace de coordination des voxels en fonction des paramètres et des positions déplacées des voxels en attribuant des coordonnées d'espaces mis à jour de voxels aux voxels, et (v) déplacer des attributs relatifs au traitement associés avec les voxels conjointement avec les voxels correspondants, (vi) modifier au moins un paramètre de traitement en fonction des nouveaux emplacements des voxels déplacés et des attributs relatifs au traitement associés avec celui-ci, le système étant utilisé pour des interventions non invasives faisant usage d'une énergie acoustique.

2. Le système selon la configuration 1, comprenant en outre un module de stockage de données pour le stockage des coordonnées d'espaces de voxels et les attributs relatifs au traitement associés avec les voxels.

3. Le système selon la configuration 1, comprenant en outre un dispositif d'imagerie permettant d'obtenir les images tridimensionnelles.

4. Le système selon la configuration 1, comprenant en outre un transducteur aux ultrasons permettant d'apporter une énergie d'ultrasons au volume de traitement.

5. Le système selon la configuration 4, comprenant en outre un contrôleur configuré pour ajuster la fourniture de l'énergie des ultrasons provenant du transducteur, en fonction des paramètres de traitement modifiés.
